# EUROPEAN PATENT APPLICATION

(11) **EP 2 927 215 A1**
(43) Date of publication of application: **07.10.2015**
(21) Application number: 15160722.3
(22) Date of filing: 25.03.2015
(51) Int. Cl.: C07D 217/04, A61P 25/28, A61K 31/472

(54) **Copper chelators**

(30) Priority: 03.04.2014 IT GE20140034
(71) Applicant: Farma-Derma S.R.L., 40010 Sala Bolognese (BO) (IT)
(72) Inventor: Russo, Vincenzo, 40126 Bologna (BO) (IT)
(74) Representative: De Anna, Pier Luigi

(57) **Abstract**

The present invention relates to a new class of chemical compounds of structure 6,7-dimethoxy-1,2,3,4-tetra-hydro-isoquinoline having therapeutic action, processes for the synthesis thereof and formulations containing said derivatives. More particularly, the present invention relates to ligand compounds adapted for chelating the free cupric ion in the serum of patients suffering from Alzheimer's and Wilson's diseases.

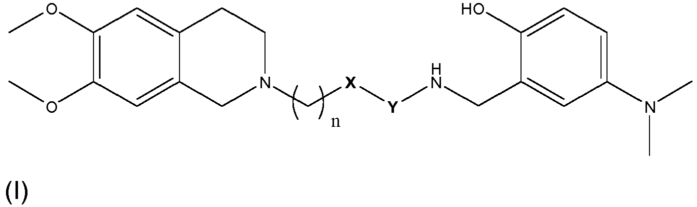

## Description

The present invention relates to a new class of chemical compounds of structure 6,7-dimethoxy-1,2,3,4 tetra-hydro-isoquinoline having therapeutic action, processes for the synthesis thereof and formulations containing said derivatives. More particularly, the present invention relates to ligand compounds capable of chelating the free cupric ion in the serum of patients suffering from Alzheimer's and Wilson's diseases.

### STATE OF THE PRIOR ART

High concentrations of free cupric ion (not ceruloplasminic) constitute a predictive factor for neurodegenerative diseases such as Alzheimer's and Wilson's disease. The copper chelators are structurally characterized by specific molecular determinants in the various molecules object of study.

The cupric ion concentration in the serum is the reference biomarker for Alzheimer's (AD) and Wilson's diseases.

New international guidelines were published in 2011 for the diagnosis of Alzheimer's disease. These guidelines deal specifically with the diagnosis of Alzheimer's disease in its earliest stages and mark a radical change in the way experts see and study Alzheimer's disease, which also include the clinical utility of incorporating tests with biomarkers.

There are currently no approved therapies for Alzheimer's disease in the prodromal phase. However, clinical trials are in progress both for potential therapies and for the early detection of the disease through the tomographic examinations and the use of biomarkers.

Alzheimer's disease is a neurological disease which involves a progressive loss of memory and cognitive functions. The diagnosis of AD is related to the response of the beta-amyloid protein (Ap) and of the tau peptides in the brain. Amyloid is produced when a protein considerably larger, the amyloid precursor, is cleaved. So the amyloid accumulates in the form of plaques (deposits) in the outer part of the nerve cells.

Amyloid deposits are considered to be toxic and cause damage to nerve cells many years before the onset of dementia. Currently there is evidence that in AD at the initial stage there are said abnormal amyloids, both in healthy individuals and in those suffering from mild cognitive decline, before they develop thereafter Alzheimer's dementia.

The second hallmark of Alzheimer's disease is the presence of tangles of a protein called tau. Although the tau protein is produced by normal, healthy nerve cells, in Alzheimer's disease an abnormal variant is produced that does not work properly. Said variant, known as p-tau, causes the formation of tangles within cells which strangle in fact neurons (nerve cells), which then die.

These two biomarkers are measurable in the cerebrospinal fluid and, in the AD, the level of beta-amyloid is less and tau peptide is increased, with respect to the control.

There are also secondary risk factors of the AD as the epsilon 4 allele of the gene of the apolipoprotein E (APO-E) (X; Toledo, et al. Neuroimage Clin. 2013, 4, 164-173). Different pathogenetic pathways that contribute to the onset of Alzheimer's disease and its progression have been theorized. Among these, the most accredited regards the formation of free copper that is unduly accumulated in the brain, causing oxidative stress facilitating the accumulation of the beta-amyloid protein. Several experimental data show that the free copper is significantly higher both in the serum of Alzheimer's patients and in subjects with AD risk, many of whom (70%) due to this high value later develop the pathology of Alzheimer's disease. (Geng, J.; Li, et al J. Med Chem. 2012, 55, 9146-9155).

Wilson's disease is linked to the absence of ceruloplasmin, a protein able to bind copper in the liver/hepatically. Right from birth, in Wilson cases, hepatic and neurological disorders can be demonstrated that may regress with appropriate therapies based on zinc salts and/or copper chelators for systemic use such as penicillamine and trientine. Copper is necessary for the organism for a number of physiological functions, it is primarily a cofactor for the proper functioning of a number of enzymes such as cytochrome-c oxidase, ceruloplasmin, dopamine β-hydroxylase, superoxide dismutase and monophenol monooxygenase.

Ceruloplasmin binds the cupric ion with a 1:6 stoichiometry representing in this form about 95% of the total copper circulating in the blood (Grazyna, G.; et al; Biometals, 2014, 27, 207-215).

The share of non-ceruloplasmin copper is responsible for neurodegenerative progression both in AD and in Wilson. The evaluation of the concentration of free copper in the serum is carried out by subtracting from the total concentration of copper, by means of atomic absorption, the concentration of ceruloplasmin analyzed by an immunoturbidimetric assay (Abe, A.; et al. Clin Chem. 1989, 35, 552-554).

WO0102366 and FR2706895 disclose substituted isoquinolines for use in the treatment of neurodegenerative diseases, including Alzheimer's disease.

Purpose of the present invention is to develop a new class of compounds to chelate copper and therefore able to subtract it from that, which is present in the bloodstream by means of a mechanism of mechanical/chemical action.

The invention is directed to nitrogen substituted isoquinoline ring compounds. There is a need of developing new effective compounds for use in the treatment of Alzheimer's disease able to chelating the copper ions. Tetrahydroisoquinoline derivatives according to the invention are effective in chelating copper ions and in the treatment of Alzheimer's disease.

### SUMMARY OF THE INVENTION

The present invention provides a new class of compounds with structure 6,7-dimethoxy-1,2,3,4- tetra-hydro-isoquinoline able to chelate the cupric ion.

The compounds according to the invention have advantageous properties in respect to stability, duration of action, and possibility of oral administration. The compounds are then eliminated from the mammalian body.

The compounds according to the invention are used for the chelation of cupric ion that accumulates in the wall of the intestinal tract of a human being and are able to subtract this ion from the wall of the intestinal tract without being absorbed into the bloodstream.

The present invention relates to compounds of formula (I) 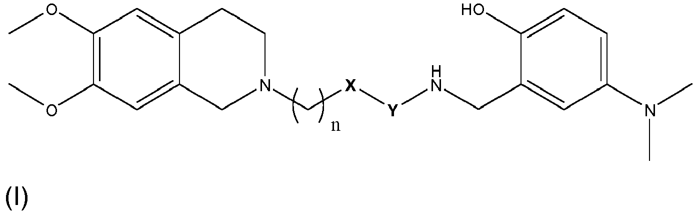 wherein
n is a C₁-C₄ alkane
and X is an aromatic ring or a corresponding hetereronucleus of formula 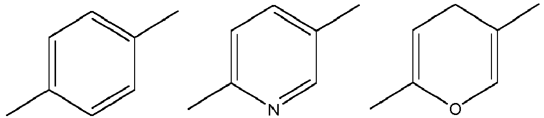 or ciclopentadienic ortho substituted of formula 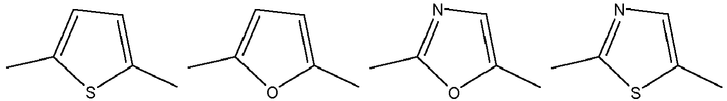 and Y is an aromatic ring or its corresponding heteronucleus of formula 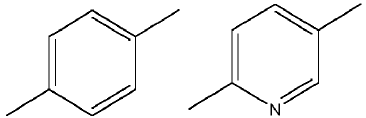 and acceptable acid salts thereof.

As examples of compounds according to the invention may be mentioned 2-((4-(6-((3,4-dihydro-6,7-dimethoxy-isoquinolin-2-(1 H)-yl)methyl)pyridin-3-yl)phenylamino)methyl)-4-(dimethylamino)phenol or 2-(((4'-((3,4-dihydro-6,7-dimethoxy-isoquinolin-2-(1 H)-yl)methyl)-(1,1'-biphenyl)-4-yl)amino)methyl)-4-(dimethylamino)phenol.

As examples of formulations containing compounds of the present invention may be mentioned formulations which can be administered orally, for example, powders for oral solutions or gelatin capsules. The content of the active compound may vary depending on the particular form of administration and can be between 0.5% and about 99% by weight per dosage unit.

The amount of active compound in said formulations is such that suitable unit doses can be obtained. Formulations and preparations according to a preferred mode of implementation of the present invention are prepared so that the oral dosage units contain from 2-20 g of active component.

The chelating agent is administered orally, through the intestinal wall, and circumvents the uptake and efflux systems on the apical membrane of these cells. In this stage, the molecule captures the copper and the chelate becomes the substrate of the intestinal extrusion systems where in free form is not extruded from said apically localized systems.

The compounds of the present invention can be synthesized according to the methods claimed in claims 6 and 7.

According to a preferred implementation method the chemical compound according to formula (I) is prepared according to a method which involves the condensation of a carboxylic acid with an isoquinoline in carbonyldiimidazole presence and formation of an amide compound, the reaction of the amide compound with an appropriate boronic acid for the formation of a nitroderivative bicyclic system; the reducing of the nitroderivative bicyclic system with a XAlH₄ and the formation of an amino derivative, the condensation with an aldehyde of the amine derivative and formation of an imine intermediate which is not isolated; and the reduction of the imine intermediate with XBH₄ for obtaining final amine, where X is an alkaline element.

According to a further preferred implementation method, the chemical compound according to formula (I) is prepared according to a method which involves the condensation of a halobenzoic acid with a nitro-aryl-boronic acid to obtain the corresponding nitroderivative; followed by condensation of the nitroderivative with an isoquinoline in the carbonyldiimidazole presence for obtaining a halogen-amide derivative; the reduction of halogen-amide compound to the corresponding amine derivative with XAlH₄, where X is an alkaline element; its condensation with an aryl-ortho-nitro-substituted aldehyde in imine intermediate; and finally the reduction of the non-isolated imine intermediate with XAlH₄, where X is an alkaline element for obtaining the final amine.

The following figure, which are included and form part of this specification, illustrate certain preferred embodiments of the invention and together with the description serve to explain the principles of the invention.

### BRIEF DESCRIPTION OF THE DRAWINGS

Figure 1 shows the spectroscopic characterization of the copper chelator (continuous line) and of the corresponding chelate (dashed line).
Figure 2 shows the example scheme of reversed intestine bioassay of a rat.

### DESCRIPTION OF THE EMBODIMENTS OF THE INVENTION

Reference to the detailed illustrative embodiments of the invention will now be made, examples of which are illustrated by the accompanying drawings and representations of the components used.

The compounds of the present invention can be prepared by two different synthetic ways according to the nature of the substituent **X** of the compound of general formula (I).

The compounds that are synthesized according to the **scheme 1** are prepared as follows: the carboxylic acid is condensed with isoquinoline in carbonyldiimidazole presence. The amide compound reacts by Suzuki reaction with the appropriate boronic acid leading to the corresponding nitroderivative bicyclic system. This intermediate is reduced with LiAlH₄ to the corresponding amino derivative and subsequently condensed with the aldehyde. The imine intermediate isolated from the reaction environment is reduced with NaBH₄ to obtain the final compound.

The synthesized compounds according to **Scheme 2** are prepared as follows: the carboxylic acid reacts with the appropriate boronic acid using Suzuki reaction to give the nitro derivative which is condensed with the isoquinoline in the presence of carbonyldiimidazole. The amide compound is reduced to the corresponding amino derivative with LiAlH₄ and then condensed with the aldehyde. The non-isolated imine intermediate is reduced with NaBH₄ to obtain the final compounds.

### Intermediates and final compounds of Scheme 1

### 1.1 Production of the intermediate

### (3,4-dihydro-6,7-dimethoxy-isoquinolin-2-(1 H) -yl)(pyridin-2-yl)_methanone(3)

5-bromo-pyridin-2-carboxylic acid (1)(1meq) is placed to react in anhydrous THF (10mL) with carbonyldiimidazole (1.1 meq) and the mixture is stirred at room temperature for five hours. 6,7-dimethoxy-1,2,3,4-tetrahydroisoquinoline (2) (1 meq) is added and it is left to react overnight. The solvent is concentrated, is recovered with water and extracted with CHCl₃ (3 x 20 mL). The compound is purified on chromatography column (CH₂Cl₂/MeOH 98/2). White solid. Yield 64% ESI ⁺/MS m/z: 399 [M ⁺Na]⁺, ESI +/MS/MS m/z: 399 (100), 227 (60). ¹H-NMR (δ):2.85(m,2H,NCH₂CH₂ isoquinoline), 3.29 (m, 2H, NCH₂CH₂ isoquinoline), 4.22 (s, 2H, NCH₂ isoquinoline), 3.78 (s, 6H, OCH₃), 6.40-9.09 (m, 5 H, aromatics).

### 1.2 Production of the intermediate

### (3,4-dihydro-6,7-dimethoxy-isoquinolin-2-(1 H)-yl)(5-(4-nitrophenyl)pyridin-2-yl) methanone (5)

4-nitrophenyl-boronic acid (4)(1 meq), bromo-amide (3) (1 meq) derivative, palladium tetrakis triphenylphosphine (0.1 meq) and a saturated solution of K₂CO₃ (3 meq) are dissolved in EtOH (15 mL) and the mixture is left to react at reflux overnight. It is filtered on Celite and the solvent is evaporated. The residue is recovered with water and extracted with CHCl₃ (3 x 20 mL). The compound is purified on chromatography column (CH₂Cl₂/MeOH 98/2). Orange solid. Yield 52% ESI⁺/MS m/z: 442 [M⁺Na]⁺, ESI⁺/MS/MS m/z: 442 (100), 339 (27). 1H-NMR(δ):2.85 (m, 2H, NCH₂CH₂ isoquinoline), 3.29 (m, 2H, NCH₂CH₂ isoquinoline), 4.22 (s, 2H, NCH₂ isoquinoline), 3.78 (s, 6H, OCH₃), 6.40-9.11 (m, 9H, aromatics).

### 1.3 Production of the intermediate

### 4-(6-((3,4-dihydro-6,7-dimethoxy-isoquinolin-2-(1 H)-yl)methyl)pyridin-3-yl)benzenamine(6)

To a suspension of LiAlH₄ (3 meq) in anhydrous THF (5 mL) is added drop by drop to the amide (5) (1 meq) previously solubilized in 5 mL of the same solvent, the reaction mixture is left at reflux for 1 h. It is concentrated and recovered with water by extracting with AcOEt (3 x 20 mL). The compound is purified on chromatography column (CHCl₃/MeOH 98/2). Orange solid. Yield 18% ESI⁺/MS m/z: 398 [M⁺Na]⁺, ESI⁺/MS/MS m/z: 240 (100), 183 (13). 1 H-NMR (δ): 2.65 (m, 2H, NCH₂CH₂ isoquinoline), 2.80 (m, 2H, NCH₂CH₂ isoquinoline), 3.62 (s, 2H, NCH₂ isoquinoline), 3.95 (s, 2H, CH₂N), 3.78 (s, 6H, OCH₃), 4 (broad s, 2H, NH₂), 6.42-8.86 (m, 9H, aromatics).

### 1.4 Production of the intermediate

### 5-(dimethylamino)-2-hydroxybenzaldehyde(7)

2-hydroxy-5-nitrobenzaldehyde (1 meq) is dissolved in 20 mL of absolute EtOH, to this mixture 5 mL of 37% acetaldehyde and the catalyst Pd/C 10% are added and the reaction is carried out under a H₂ atmosphere leaving to react at room temperature for 24 h. It is filtered on Celite, the solvent is concentrated to obtain a yellow solid. Yield 80%. ESI⁻/MS m/z: 164 [M-H]⁻, ESI-/MS/MS m/z: 149 (20),121 (5).

### 1.5 Production of the compound according to the invention

### 2-((4-(6-((3,4-dihydro-6,7-dimethoxy-isoquinolin-2-(1 H)-yl)methyl)pyridin-3-yl)phenylamino)methyl)-4-(dimethylamino)phenol.(8)

The amine (6) (1 meq) and the aldehyde (7) (3 meq) are solubilized in 10 mL of anhydrous CH₂Cl₂ in the presence of catalytic amounts of para-toluenesulfonic acid and molecular sieves. It is left to reflux overnight. The imine obtained is not isolated and, after evaporation of the solvent, is solubilized in 10 mL of MeOH and reduced in the presence of NaBH₄. MeOH is evaporated, recovered with water and extracted with CHCl₃ (3 x 20 mL). The compound is purified on chromatography column (CHCl₃). Yellow solid. ESI⁺/MS m/z: 547[M⁺ Na]⁺, ESI⁺/MS/MS m/z: 301 (100), 547 (23). 1 H-NMR (δ): 2.65 (m, 2H, NCH₂CH₂ isoquinoline), 2.69 (m, 2H, NCH₂CH₂ isoquinoline), 2.85 (m, 6H, NCH₃), 3.62 (s, 4H, NCH₂ isoquinoline and CH₂N), 3.78 (s, 6H, OCH₃), 4 (broad s, 1 H, NH), 4.32 (s, 2H, NHCH₂), 5 (s, 1 H, OH) 6.22-7.36 (m, 12 H, aromatics).

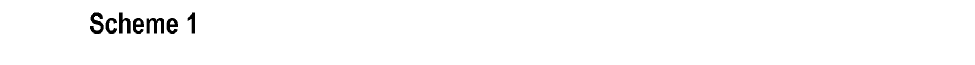

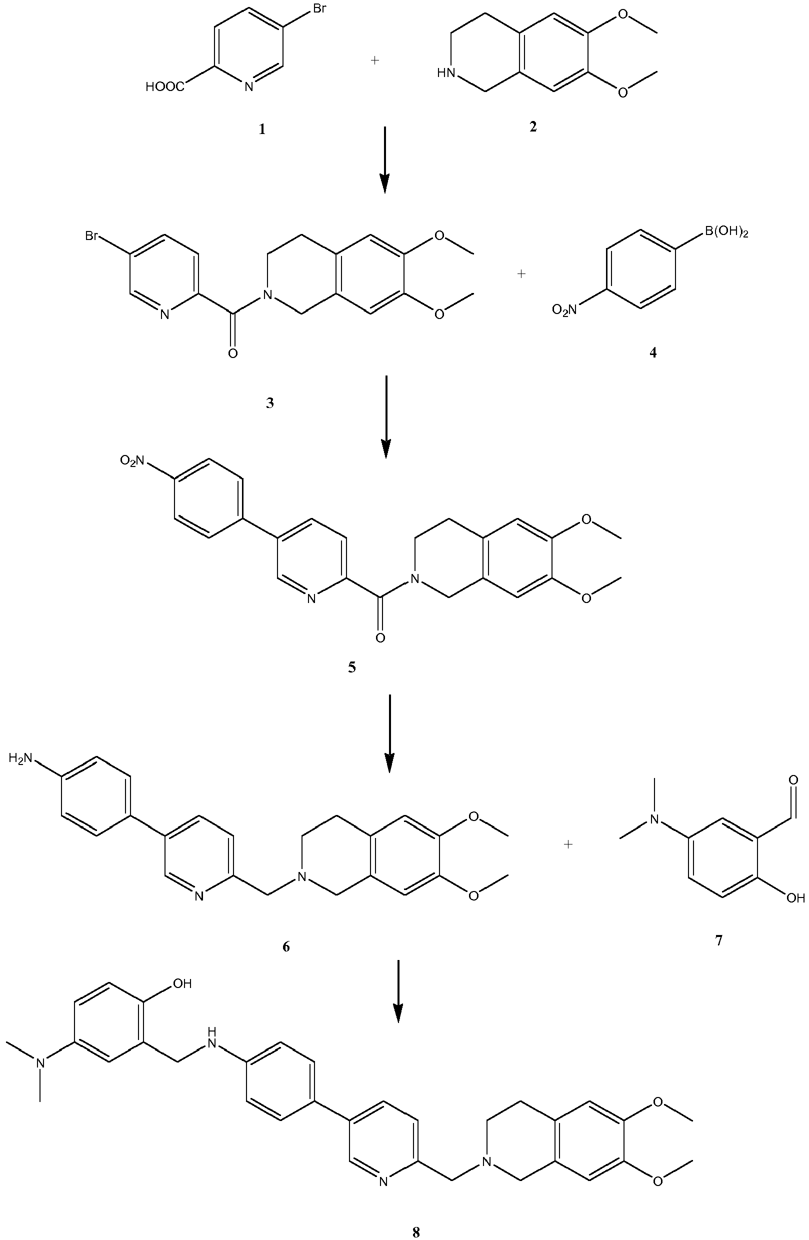

### Intermediates and final compounds of Scheme 2

### 2.1 Production of the intermediate

### 4'-Nitro-(1,1'-biphenyl)-4-carboxylic acid (11)

4-nitrophenyl-boronic acid (9) (1.5 meq), 4-bromo-benzoic acid (10) (1 meq), tetrakis triphenylphosphine palladium (0.05 meq) and a 2 M solution of NaHCO₃ (3 meq) are dissolved in 1,4-dioxane (20 mL) and the mixture is left to react at reflux overnight. The solvent is concentrated, recovered with CHCl3 (20 mL) and with water. The aqueous phase is acidified (HCl 6 N 20 mL) giving a precipitate which is filtered and left to dry. White solid. Yield 76% ESI⁻/MS m/z: 242 [M-H]⁻, ESI⁻/MS/MS m/z: 198 (100), 168 (7). 1 H-NMR (δ): 7.74-8.25 (m, 8H, aromatics), 11 (s, OH).

### 2.2 Production of the intermediate

### (3,4-dihydro-6,7-dimethoxy-isoquinolin-2-(1 H) -yl)-(4'-nitro-(1,1'-biphenyl)-4-yl) methanone (12)

The carboxylic acid previously described (11) (1 meq) is placed to react in anhydrous THF (20 mL) with carbonyldiimidazole (1.1 meq) and the mixture is stirred up at room temperature for five hours. 6,7- dimethoxy-1,2,3,4-tetrahydroisoquinoline(2) (1 meq) is added and it is left to react overnight. It is concentrated, is recovered with water and extracted with CHCl₃ (3 x 20 mL). The compound is purified on chromatography column (CHCl₃).Yellow solid. Yield 55% ESI⁺/MS m/z: 441 [M⁺ Na]⁺, ESI⁺/MS/MS m/z: 441 (100). 1 H-NMR (δ): 2.90 (m, 2H, NCH₂CH₂ isoquinoline), 3.78 (s, 6H, OCHs), 4.15 (m, 2H, NCH₂CH₂ isoquinoline), 4:56 (s, 2H, NCH₂ isoquinoline) 7.57-8.35 (m, 10H, aromatics).

### 2.3 Production of the intermediate

### 4-((3,4-dihydro-6,7-dimethoxy-isoquinolin-2-(1H)- il) methyl)- 1,1'-biphenyl)-4-amine (13)

To a suspension of LiAlH₄ (3 meq) in anhydrous THF (5 mL) is added drop by drop to the amide (12) (1 meq) solubilized in 5 mL of THF leaving all at reflux for two hours. Is concentrated and the residue is recovered with water and extracted (3 x 20 mL) with CHCl₃. The mixture is purified on chromatography column (CHCl₃).

Yellow solid. Yield 40% ESI+/MS m/z: 375 [M⁺ H]⁺, ESI⁺/MS/MS m/z: 182 (100). 1 H-NMR (δ): 2.65 (m, 2H, NCH₂CH₂ isoquinoline), 2.80 (m, 2H, NCH₂CH₂ isoquinoline), 3.62 (s, 4H, NCH₂ isoquinoline and CH₂N), 3.78 (s, 6H, OCH₃), 4 (broad s, 2H, NH₂), 6.42-7.35 (m, 10H, aromatics).

### 2.4 Production of the intermediate

### 5-(dimethylamino)-2-hydroxybenzaldehyde (7)

2-hydroxy-5-nitrobenzaldehyde (1 meq) is dissolved in 10 mL of absolute EtOH; to this mixture are added 5 mL of acetaldehyde 37% and the catalyst Pd/C 10%. The reaction is carried out under a H2 atmosphere leaving to react at room temperature for 24 h. The mixture is filtered over Celite and the solvent is concentrated. Yellow solid. Yield 80%. ESI⁻/MS m/z: 164 [M-H]⁻, ESI⁻/MS/MS m/z: 149 (20), 121 (5).

### 2.5 Production of the compound according to the invention

### 2-(((4'-((3,4-dihydro-6,7-dimethoxy-isoquinolin-2-(1 H)-yl)methyl)-(1,1'-biphenyl)-4-yl)amino)methyl)-4-(dimethylamino)phenol. (15)

The amine (13) (1 meq) and the aldehyde (7) (1.5 meq) are reacted overnight in anhydrous toluene (10 mL) in the presence of catalytic amounts of para-toluenesulfonic acid. The imine obtained is not isolated and, after evaporation of the solvent, is solubilized in 10 mL of MeOH and reduced in the presence of NaBH₄. MeOH is evaporated, recovered with water and extracted with CHCl3 (3 x 20 mL). The compound is purified on chromatography column (CHCl₃). Yellow solid. Yield 18% ESI⁻/MS m/z: 522 [M-H]⁻, ESI⁻/MS/MS m/z: 373 (100). 1 H-NMR (δ): 2.65 (m, 2H, NCH₂CH₂ isoquinoline), 2.80 (m, 2H, NCH₂CH₂ isoquinoline), 2.85 (m, 6H, NCHs), 3.62 (s, 4H, NCH₂ isoquinoline and CH₂N), 3.78 (s, 6H, OCH₃), 4 (broad s, 1 H, NH), 4.32 (m, 2H, NHCH₂), 5 (s, 1 H, OH) 6.22-7.36 (m, 13 H, aromatics).

The compounds according to the invention are prepared according to the scheme 1 that according to the scheme 2 have ligand characteristics of Cu⁺⁺ ion. To this purpose, the compounds according to the invention were studied *in vitro* and *ex vivo* for their ability to chelate copper (constant of formation, K_{f} and metabolic stability).

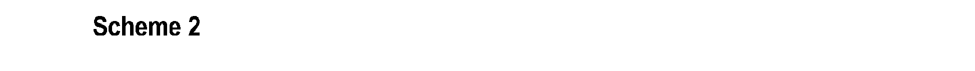

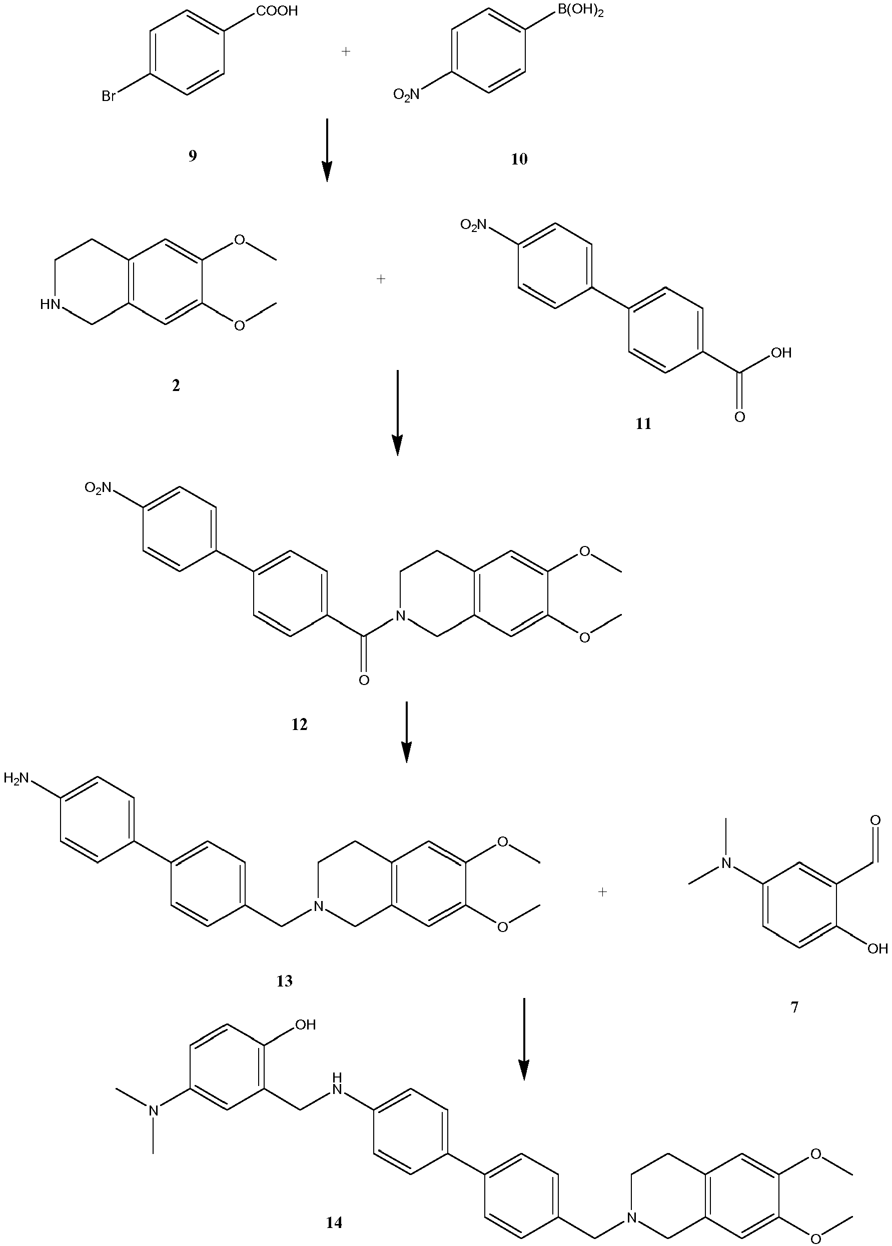

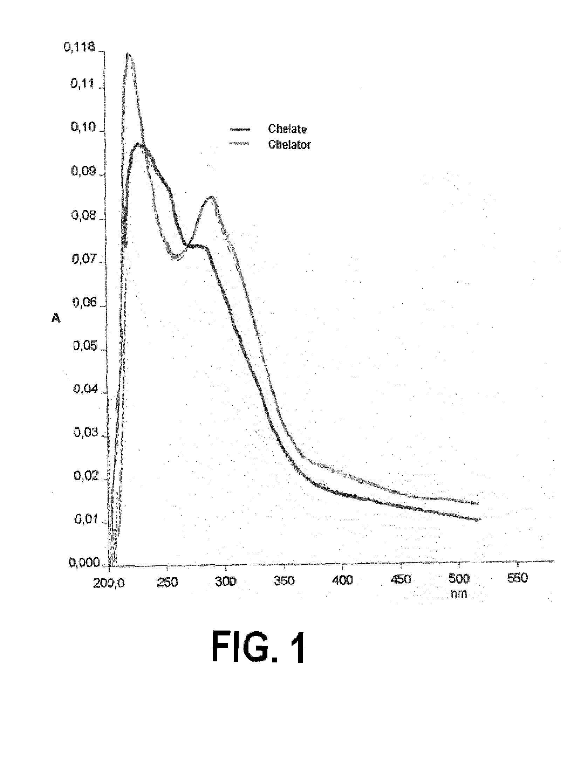

To this purpose the spectroscopic characterization of both the chelating agent and the chelate was obtained as reported in Figure 1 and a calibration curve by means of studies of UV-Vis spectrophotometry (Lambda Bio-20, Perkin-Elmer) which highlight the formation of the chelate as a function of the concentration of the free cupric ion, of the solvent and of the pH.

After determining the chemical and physical-chemical parameters of some compounds adapted to chelate copper we moved to a further evaluation *in vitro* using sera of Alzheimer's patients in which the concentration of free cupric ion resulted high. These samples, studied in parallel with an untreated sample, were added with the chelating agent at a concentration previously established (10⁻⁴ M in the sample). The concentration of free cupric ion in the serum of healthy subjects is between 0.1 µM and 1.6 µM. Therefore, taking into account the constant formation (K_{f}), to a value of [Cu⁺⁺] = 1.6 µM the formation of the chelate is not appreciable. In other words, the concentration of the chelating agent (10⁻⁴ in the sample) is chosen so as to subtract Cu⁺⁺only when the concentration of the same is > 1.6 µM.

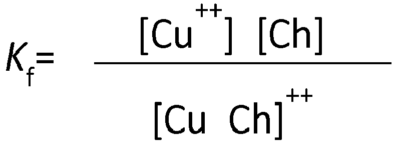

Chelates with zinc have also been studied which show constant formations higher than those with copper: this aspect can have a double meaning.
1. The compound may be administered as a zinc chelate which in vivo is moved from the copper. Zinc is released. This fact is very important also in terms of therapy against Alzheimer's disease both because it competes with the copper but with less neurotoxicity for the transport sites through the BBB and because zinc stimulates metallothioneins, endogenous proteins adapted to chelate copper;
2. The reduced ability to chelate copper with respect to zinc constitute in vivo the selectivity of the chelating agent proposed.

After addition of the chelating agent the percentage amount of copper removed from serum by way of chelation is evaluated. In Table 1 are shown the variations of concentration of cupric ion in the presence of the chelating agent on selected samples in a clinical setting having high value of free cupric ion (normal range 0.1 µM - 1.6 µM)

**Table 1. Alzheimer's samples**

| Patient ID | Serum not treated with chelating agent [Cu++]µM | Serum treated with chelating agent [Cu++]µM | Variation % |
|---|---|---|---|
| **1 (D. C.)** | 2.5 | 2.35 | -6 |
| **2 (C. O.)** | 2.6 | 2.2 | -15 |
| **3 (I. C.)** | 2.6 | 2.1 | -20 |
| **4 (F. F.)** | 1.9 | 1.4 | -30 |
| **5 (S. M.)** | 1.9 | 1.6 | -16 |
| **6 (B. L.)** | 1.9 | 0.8 | -58 |
| **7 (N. P.)** | 2.5 | 2.15 | -14 |
| **8 (P. C.)** | 5.2 | 3.4 | -35 |
| **9 (C. N)** | 1.8 | 1.55 | -14 |
| **10 (Z. L.)** | 2.05 | 1.7 | -17 |
| **11 (C. M)** | 1.75 | 0.65 | -63 |
| **12 (F. A.)** | 1.85 | 1.65 | -10 |
| **13 (N. P.)** | 2.4 | 2.0. | -15 |
| **14. (B. I.)** | 2.2 | 1.55 | -30 |
| **15. (B. F.)** | 1.85 | 1.6 | -13 |
| **16 (D. F.)** | 2.05 | 1.8 | -12 |
| **17 (D. V.)** | 1.8 | 1.5 | -16 |

In control samples (healthy subjects) is proceeded similarly achieving no appreciable changes in concentration as shown in Table 2.

**Table 2. Control sample**

| Patient ID | Serum not treated with chelating agent [Cu⁺⁺]µM | agent [Cu⁺⁺]µM |
|---|---|---|
| **1 (S.I.)** | 0.35 | 0.30 |
| **2 (A. A.)** | 0.55 | 0.55 |
| **3 (C. M.)** | 1.45 | 1.5 |
| **4 (G. S.)** | 1.25 | 1.55 |
| **5 (M. S.)** | 1.1 | 1.55 |
| **6 (F. C.)** | 1.5 | 1.45 |
| **7 (F. R.)** | 1.6 | 1.5 |

The results show that for Alzheimer subjects ([Cu⁺⁺] > 1.6 µM) there is formation of the chelate which then subtracts cupric ion from the serum. The percentage change of cupric ion in the serum of AD patients does not depend on the concentration of copper in the serum of the patient, but probably by other subjective factors such as pH and the presence of other strong electrolytes. In the control samples instead it is noted that the chelating agent, as expected, is unable at that concentration to further subtract cupric ion, and this provides a safety margin in terms of excessive depletion of cupric ion in the organism.

The metabolic stability and pharmacokinetics in the reversed intestine of rats were also explored.

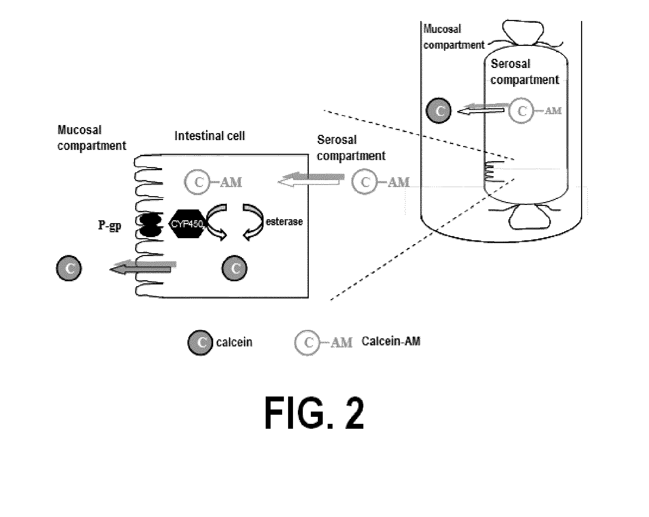

The method of rat reversed organ allows to determine whether a compound is able to cross the intestinal mucosa and simultaneously how much the intestinal enzymatic activity affects the activity of the compound in question. The results show that the copper chelators belonging to class 6,7-dimethoxy-1,2,3,4- tetra-hydro-isoquinoline are able to permeate the intestinal wall and to chelate the cupric ion if the concentration thereof is > 10⁻⁶ M. Subsequently they are actively transported out of the cellular compartment by efflux mechanisms localized on the apical membrane. From the point of view of metabolic stability the chelating agent is stable for 60 minutes (90%) and after two hours metabolites are present that are not absorbed but that are actively transported by efflux systems localized in the apical portion. This bioassay is shown schematically in Figure 2 with the fluorescent labels used to study the transport and the metabolism (Calcein-AM).

The spectroscopic properties of the final compounds were determined by UV-Vis analysis at different concentrations (range from 10⁻³ M to 10⁻⁶ M) and in different solvents (methanol, acetonitril and PBS at various pH). The best experimental conditions have been obtained at a ligand concentration equal to 10⁻⁵, in PBS at pH 7.9.

The ability of the compound to chelate the cupric ion was studied in UV-Vis spectroscopy. The absorption spectrum was recorded for the individual ligand (10⁻⁵M in methanol) and after the addition of a 10⁻² solution of CuCl₂ to the ligand. Note the variation of both the maximum wavelength of absorption and of the absorbance due to the formation of the complex ligand-Cu⁺⁺.

The constant formation *K*_{f} was determined for the compound according to the invention: 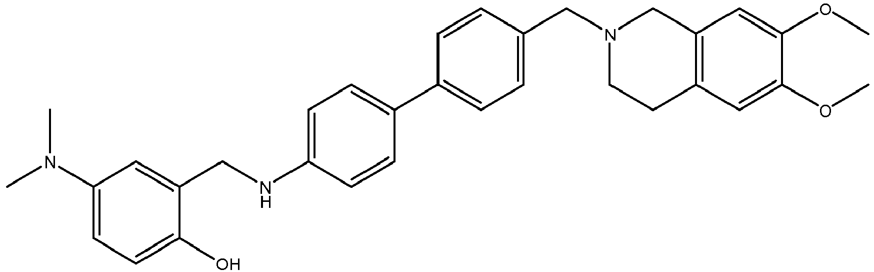 *K*_{f}= 4,2 x 10²

## Claims

1. A chemical compound of structure 6,7-dimethoxy-1,2,3,4-tetra-hydro-isoquinoline, according to formula **(I)** 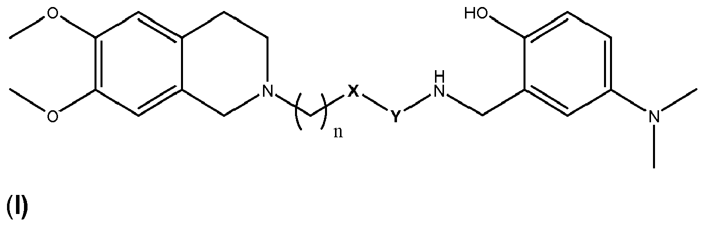 wherein
n is a C₁-C₄ alkane
and X is an aromatic ring or a corresponding hetereronucleus of formula 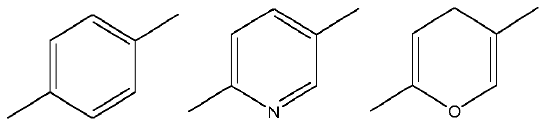 or ciclopentadienic ortho substituted of formula 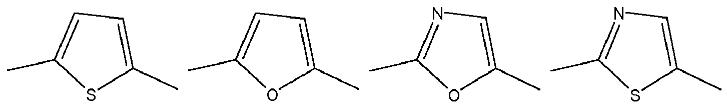 and Y is an aromatic ring or its corresponding heteronucleus of formula

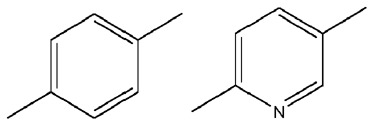

2. The chemical compound according to claim 1, wherein n =C₁

3. The chemical compound according to claims 1 and 2, wherein X is an aromatic ring of formula 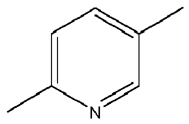 and Y is a benzene ring of formula

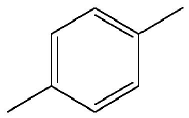

4. The chemical compound according to claims 1 and 2, wherein X is a benzene ring of formula 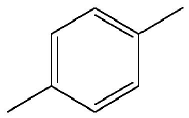 and Y is a benzene ring of formula

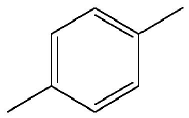

5. The chemical compound according to claims 1-4, wherein the chemical compound is a metal chelate, preferably a zinc chelate.

6. A preparation method of the chemical compound according to formula **(I)**, comprising the following steps:
a. Condensation of a carboxylic acid with an isoquinoline in carbonyldiimidazole presence and formation of an amide compound;
b. Reaction of the amide compound with a suitable boronic acid for the formation of a nitroderivative bicyclic system;
c. Reduction of the nitroderivative bicyclic system with a XAlH₄ and formation of an amine derivative;
d. Condensation of the amine derivative with an aldehyde and formation of an imine intermediate which is not isolated;
e. Reduction of the imine intermediate with XBH₄ for obtaining final amine; wherein X is an alkali element.

7. The preparation method of the chemical compound according to formula **(I),** comprising the following steps:
a. Condensation of a halobenzoic acid with a nitro-aryl-boronic acid to obtain the corresponding nitroderivative;
b. Condensation of the nitroderivative of step a) with an isoquinoline in carbonyldiimidazole presence to obtain a halogen-amide derivative;
c. Reduction of the halogen-amide compound to the corresponding amine derivative with XAlH₄, where X is an alkali element;
d. Condensation of the amino derivative with an aryl-ortho-nitro-substituted aldehyde in imine intermediate;
e. Reduction of the not isolated imine intermediate with XAlH₄, where X is an alkali element for obtaining the final amine.

8. A compound according to claims 1-5 for medical use.

9. The compound according to claims 1-5 for use in a method for the treatment of Alzheimer's disease.

10. The compound according to claims 1-5 for use in a method for the treatment of Wilson's disease.

11. A formulation comprising at least one compound according to claim 1, or a therapeutically acceptable salt thereof, and an acceptable excipient.
